Europäisches Patentamt

European Patent Office

Office européen des brevets

(19)

(11) Publication number : **0 535 804 A1**

# (12) EUROPEAN PATENT APPLICATION

(21) Application number : **92307976.8**

(22) Date of filing : **03.09.92**

(51) Int. Cl.[5] : **C07K 1/00,** C07K 7/06,
G01N 33/74, A61K 37/02,
A61K 37/43, C12Q 1/68

(30) Priority : **03.09.91 JP 221971/91**

(43) Date of publication of application :
**07.04.93 Bulletin 93/14**

(84) Designated Contracting States :
**DE FR GB IT**

(71) Applicant : **HITACHI CHEMICAL CO., LTD.**
**2-1-1, Nishishinjuku**
**Shinjuku-ku, Tokyo (JP)**

(72) Inventor : **Hayashi, Takashi**
**3-4-1, Matsushino**
**Tsukuba-shi, Ibaraki-ken (JP)**

Inventor : **Watanabe, Hiroo**
**1-2-2, Benten-cho**
**Hitachi-shi, Ibaraki-ken (JP)**
Inventor : **Izutsu, Hiroshi**
**1596-10 Maeno**
**Tsukuba-shi, Ibaraki-ken (JP)**
Inventor : **Odagawa, Yasuhisa**
**Yamazaki-ryo 139, 4-2-9, Higashi-cho**
**Hitachi-shi, Ibaraki-ken (JP)**
Inventor : **Bamba, Kenzou**
**3-4-3, Matsushiro**
**Tsukuba-shi, Ibaraki-ken (JP)**

(74) Representative : **Holmes, Michael John et al**
**Frank B. Dehn & Co. European Patent**
**Attorneys Imperial House 15-19 Kingsway**
**London, WC2B 6UZ, (GB)**

(54) **Peptide compounds.**

(57)    A method for designing peptides having an activity to bind to a biologically active peptide wherein an amino acid sequence of the receptor of a biologically active peptide, and an amino acid sequence obtained by deducing a complementary DNA strand of a DNA strand comprising a genetic code able to code for said biologically active peptide are compared, and a partial sequence of the amino acid sequence of said receptor is selected based on the homology of both amino acid sequences. The peptides so produced may be used diagnostically or in medicine by binding to the biologically active peptide.

EP 0 535 804 A1

## Background of the Invention

### 1. Field of the Invention

This invention relates to a method for designing peptides having an activity to bind to a biologically active peptide.

More particularly, it relates to a method for designing said peptides wherein an amino acid sequence of a receptor of a biologically active peptide, and an amino acid sequence deduced from complementary stranded DNA to a DNA strand comprising a genetic code able to code for said biologically active peptide are compared, and a partial amino acid sequence of said receptor is selected based on the homology of both amino acid sequences.

It also relates to said peptides and a process for producing the same.

It further relates to a method for determining a binding site of the receptor. The present peptides eliminate instability and fortuity of pharmaceutical drugs from biological sources while maintaining biological specificity and biological affinity.

The present peptides include fundamental capabilities of drugs from biological sources, such as specificity and affinity. In addition; they can also be used as developmental alternative drugs for current diagnostics or reagents or biochemical reagents in that they eliminate instability of drugs from biological sources being organic high molecular weight compounds. Moreover, they can also be used as medicines, such as drugs preventing the pathological disorder caused by increases of an amount of a biologically active peptide and/or activities, or therapeutic drugs that suppress or inhibit the biological effects of said biologically active peptide.

### 2. Description of the Prior Art

Affinity that exists between two molecules participating in a specific reaction carried out in a living body, such as a hormone and its receptor, antigen and antibody or enzyme and substrate, is related to a primary sequence both molecules or higher order conformational structure of the constituent amino acids. However, due to the difficulty in identifying specific binding sites, efforts to identify those sites were forced to rely upon molecules having altered sites by substitution or deletion based on experience and estimates, or design of site specific labels. With respect to the identification of the hormone binding site of a receptor, efforts were made to limit said binding site by site modification and site-specific labelling based on estimates of a secondary structure of receptor protein, protease digestion experiments and speculates from binding site sequences in other receptors. In addition, similar efforts were also made with respect to identification of substrate binding sites of enzymes. On the other hand, with respect to identification of antigen binding sites of antibodies, although it has been proven from analysis of structural genes coding for antibody molecules that amino acid sequences of variable regions determine specificity of said binding site with respect to an antigen, attempts to identify the binding site have been limited to only some antibody molecules.

In recent years, a concept has been advocated by Blalock et al. in which an amino acid sequence that constitutes a hormone binding site of a peptide hormone receptor is positioned as a translation product that has passed through mutations during a course of evolution, originating in a translocation of a complementary stranded DNA strand of a DNA sequence coding for said peptide hormone (Proc. Natl. Acad. Sci. U.S.A., 82, 1372-1375, 1985). Then, based on said concept, it means that the peptide specified by the complementary strand of the DNA sequence coding for the peptide hormone possesses binding activity to its original peptide. In fact, it has been proven that there is affinity between adrenocorticotropic hormone and the peptide specified by the complementary strand of the DNA coding for said hormone (Proc. Natl. Acad. Sci. U.S.A., 82, 1372-1375, 1985).

According to the concept advocated by Blalock et al., the receptors of biologically active peptides represented by hormones are positioned as functional transcription translation products that occur as a result of mutation during the course of evolution of the complementary strand of DNA coding for biologically active peptides. According to the principle of natural selection, genetic mutations occur in the direction in which genetic information is coded that produces biomolecules having greater function. Thus, although it is possible to design peptides having binding reactivity using the "method of designing complementary polypeptides" similarly published by Blalock et al., in which peptides are stipulated by the complementary DNA strand able to code for a biologically active peptide (Japanese Patent International Publication No. 62-502513, PCT/US86/0053), it is difficult to design the partial sequences of those peptides having adequate binding reactivity. In addition, it is also difficult to obtain peptides having a high degree of binding activity in a manner of biological components such as hormone binding sites of receptors.

Detailed Description of the Invention

The invention is concerned with a method for designing a peptide having an activity to bind to a biologically active peptide (Peptide A) which comprises the steps of:

a) determining an amino acid sequence (Sequence R) of the receptor of Peptide A;

b) deducing a DNA strand (Strand C) complementary to a DNA strand comprising genetic codons able to code for Peptide A;

c) determining an amino acid sequence (Sequence C) of a peptide complementary to Peptide A based on Strand C;

d) comparing homology of Sequence R and Sequence C; and

e) selecting from Sequence R or Sequence C the partial amino acid sequence consisting of at least more than 6 amino acid residues which a number of matching amino acids in the same order is equal to or greater than a number of not matching amino acids.

The invention also relates to a method for producing a peptide having an activity to bind to Peptide A which comprises designing a peptide by said method and producing a peptide containing a consecutive sequence of at least 4 amino acids in the designed peptide.

The invention relates to a peptide having an activity to bind to Peptide A or a salt thereof produced by said method.

The invention further relates to a method for determining a binding site of Peptide A on a receptor of Peptide A which comprises the steps of:

a) determining an amino acid sequence of the receptor of Peptide A;

b) deducing a DNA strand complementary to a DNA strand comprising genetic codons able to code for Peptide A;

c) determining an amino acid sequence of a peptide complementary to Peptide A based on Strand C;

d) comparing homology of Sequence R and Sequence C; and

e) selecting from Sequence R the partial amino acid sequence consisting of at least more than 6 amino acid residues in which a number of matching amino acids in the same order is equal to or greater than a number of not matching amino acids.

The invention relates to a diagnostic reagent for detecting Peptide A containing said peptide.

In the present invention, the method of Blalock (Japanese Patent International Publication No. 62-502513, PCT/US86/00353) can be used for the method for obtaining an amino acid sequence by deducing the complementary stranded DNA to a DNA strand comprising a genetic code able to code for a biologically active peptide. In other words, after the sequence of the gene able to code for a biologically active peptide is firstly designed and then the complementary DNA strand is designed, a complementary peptide for the whole amino acid sequence of the above-mentioned biologically active peptide is designed according to the method of Blalock by deducing the genetic codon constituting said complementary DNA strand from the 5′ or 3′ direction and orienting from the N terminus or C terminus of the peptide. Although four types of complementary peptides can be designed depending on the direction of deducing of the above-mentioned genetic codon and the direction of orientation of the peptide, since there is more than one gene sequence able to code for a biologically active peptide, a plurality of complementary peptides are obtained by four different design methods. However, it is preferable to use the sequences of all of the complementary peptides obtained for the next step (step d).

Next, the homology between the sequence of said designed peptide and the receptor of a biologically active peptide is examined. A preferable method used for implementing evaluation standards with respect to whether the amino acid sequence of the receptor of a biologically active peptide is homologous with the amino acid sequence obtained by deducing the complementary DNA strand as indicated above involves selecting those sequences as being homologous in the case where a number of matching amino acids in the same order is equal to or greater than a number of not matching amino acids, when designing all of the partial sequences composed by fixed number of six amino acid residues or more by moving one amino acid residue at a time from one of the terminuses of each of the peptide sequences, and then comparing those partial sequences. Here, there is no limitation on the number of amino acids of the partial sequences as long as they are in the form of six consecutive residues. This is because the mathematical probability that a sequence of six amino acids is specific for a biologically active peptide is one-sixty-four millionth, which is an adequate value in terms of discussing biological probability and specificity.

Although the peptides that are selected according to this method may be partial sequences of the receptor peptide side, or partial sequences of the amino acid sequence (complementary peptide) obtained by deducing of the complementary DNA strand, it is preferable to select partial sequences on the receptor side having a high degree of the activity to bind.

Moreover, in this case, it is preferable to select peptides found in partial sequences of regions located mainly on the cell surface of the receptor of a biologically active peptide, and particularly preferable to select partial sequences having a β-turn or coil structure for the receptor structure, as well as partial sequences that are not binding sites of sugar chains.

In the case where there are numerous peptides selected according to the method described above, it is preferable to make selections by limiting those peptides having matching consecutive amino acid sequences functioning as homologous sites. Moreover, it is particularly preferable to select those peptides having a larger number of matching consecutive amino acid sequences.

In addition, as another means of handling such cases, design can be performed by limiting those genetic codes able to code for a biological peptide to genetic codons that actually code for said biologically active peptide.

Moreover, the selection of partial sequences selected with at least two types of methods after designing using the above-mentioned four design methods, and more preferably, selection of partial sequences selected with as many methods as possible, is preferable since it is likely that such partial peptides will have a higher degree of binding reactivity.

In addition, peptides designed according to any of the methods described in the above-mentioned methods in which the N terminus and C terminus of the amino acid sequence of said peptides is reversed can also be selected as a peptide having an activity to bind to a biologically active peptide.

The method of designing peptides of the present invention as described above can be applied to various biologically active peptides as long as the amino acid sequence of the biologically active peptide and the amino acid sequence of its receptor are known. Some examples of such biologically active peptides include endothelin, diuretic hormone, interleukin, angiotensin, substance P and intestinal blood vessel activating peptide, although not limited to these.

A peptide is produced from a peptide sequence designed in the manner described above that contains a consecutive sequence of at least 4 amino acids of said peptide sequence that actually possesses an activity to bind to a biologically active peptide. This is because the mathematical probability of a sequence of 4 amino acids being specific for a biologically active peptide is one-sixteen hundredth, which is an adequate value for discussing biological specificity.

On the other hand, a number of amino acids constituting the peptides produced by the present method is preferably of less than about two-third of a number of the amino acids constituting the peptide having a biological activity considering the balance between a binding activity and easiness of treatment. Specifically, a number of less than 15 of amino acids is preferable.

Production of peptides can be performed by known methods, for example, by chemical synthesis using the solid phase and liquid phase methods, or by biosynthesis, etc. in which a DNA vector containing a DNA strand able to code for said peptide is prepared, and after transforming a cultured procaryotic or eucaryotic cell by said DNA vector, the peptide is produced from the transformed cell.

The following list specific examples of peptides designed and produced in the manner having a high degree of an activity to bind to and specific reactivity with a biologically active peptide.

(1) In the Case of Biologically Active Peptide being Endothelin

Peptides having at least 4 consecutive amino acid sequences selected from each of the amino acid sequences of TKITSA, VFLCKL, KGAQHR, ASTIKT, LKCLFV AND RHQAGK originating in bovine type A receptor;

Peptides having at least 4 consecutive amino acid sequences selected from each of the amino acid sequences of KRGFPP, AEVTKGGRVA, GAEMCK, SDYKGK, KTAFMQ, REVAKTVF, FVTKAVER, QMFATK, KGKYDS, KCMEAG, AVRGGKTVEA and PPFGRK originating in rat type B receptor; and,

Peptides having at least 4 consecutive amino acid sequences selected from each of the amino acid sequences of DVTKMQT and TQMKTVD originating in complementary peptide.

(2) In the Case of Biologically Active Peptide being Diuretic Hormone

Peptides having at least 4 consecutive amino acid sequences selected from each of the amino acid sequences of GVCSDTAA, TAGAPA, SPDAFR, EAGLCG, GCLGAE, RFADPS, APAGAT and AATDSCVG originating in type A receptor;

Peptides having at least 4 consecutive amino acid sequences selected from each of the amino acid sequences of REPPNP, DPSCDK, KDCSPD and PNPPER originating in type B receptor; and,

Peptides having at least 4 consecutive amino acid sequences selected from each of the amino acid sequences of VRPAIEYALR and RLAYEIAPRV originating in type C receptor.

In particular, those peptides originating in type C receptor demonstrated an especially high degree of binding activity.

Additionally, the peptides optionally blocked or protected in N terminus and/or C termius, i.e., the peptides bound to other substance, are also included in the present invention.

The salts composed of ions that are accepted to be used for pharmaceutical application, diagnostic reagents or regulation of substances of biological origin of the peptides produced in the manner described above are also included in the present invention.

Examples of such salts include acid addition salts and basic salts, while specific examples include acid addition salts with inorganic acids such as hydrochloric acid, sulfuric acid, phosphoric acid and pyrophosphoric acid; and with organic acids such as acetic acid, lactic acid, palmitic acid, stearic acid, propionic acid, citric acid, tartaric acid, malic acid, ascorbic acid, oxalic acid, methanesulfonic acid, benzenesulfonic acid and p-toluenesulfonic acid; alkali metal salts such as sodium salts and potassium salts; alkaline earth metal salts such as calcium salts and magnesium salts; ammonium salts; and, triethylamine salts.

In the case of using peptide or its salt designed according to the present invention in a pharmaceutical application, the prescribed amount of that peptide or salt that is either equal to or less than the effective amount is formed into a preparation. This preparation can be in the form of a powder, elixir, solution, pill, capsule, small pill, tablet, spray or inhalant that contains a pharmaceutically acceptable carrier. Examples of carriers that are allowed to be used for this purpose include starch, sugar, protein, talc, conventionally used synthetic rubber or natural rubber and water. Moreover, although there are no particular limitations on the administration route of the pharmaceutical preparation, examples of such include subcutaneous, intradermal, intramuscular and intravenous injections, optionally injection into the cerebrospinal lumen and administration via a mucous membrane such as, oral administration, percutaneous administration, suppositories, sprays or eye drops.

Moreover, as the peptides designed according to the present invention have binding activity to the biologically active peptide on which said design is based, they can also be applied as ligands of carriers for affinity chromatography used during purification of said biologically active peptide or its precursors. In addition, since peptides designed according to the present invention are peptide sequences containing ligand binding sites of the receptor for the biologically active peptide on which said designed is based, antibodies to the designed peptide can also be used as ligands of carriers for immunochromatography during purification of biologically active peptide receptors. Moreover, said peptides can also be used as neutralizing antibodies for receptor reactions.

In the case of obtaining an antibody, the peptide can be used as an immunogen by binding to a high molecular weight protein or functional high molecular weight compound. Examples of high molecular weight proteins able to bind said peptide include shellfish hemocyanin, bovine serum albumin, bovine thyroglobulin and avian gamma-globulin, while examples of functional high molecular weight compounds include polyvinyl pyrrolidone. However, said high molecular weight proteins and functional high molecular weight compounds are not limited to the above examples.

Further, said peptide or its salt can be used as a diagnostic reagent by binding a prescribed amount of said peptide or its salt to an enzyme for detection, substance for detection or carrier for immobilization. Examples of enzymes for detection include horseradish peroxidase, alkaline phosphatase, glucose oxidase, glucose hexaphosphate dehydrogenase and galactosidase, while examples of substances for detection include radioisotopes such as $^{125}I$, fluorescent substances such as FITC, and magnetized dextran. However, said enzymes and substances are not particularly limited to these examples. Examples of carriers for immobilization include latex particles, agarose resin, acrylamide resin, polystyrene resin and polyethylene resin, although not restricted to the above. Moreover, in the case of binding said peptide to an enzyme for detection, substance for detection or carrier for immobilization, spacer structures may be placed between these substances and the peptide by means of covalent bonds and so forth.

In addition, the method of designing peptide sequences based on the present invention can be used as a method for determining the ligand binding site of the receptor of a biologically active peptide.

Moreover, in this case, a peptide having binding reactivity to said partial sequence of the receptor can be designed according to the method of Blalock (Japanese Patent International Publication No. 62-502513, PCT/US86/0053) based on the amino acid sequence identified by the method of the present invention. Peptides obtained in this manner function as antagonists or activators of biologically active peptides. Moreover, these antagonistic or activating peptides also can be used as leading compounds when designing more sophisticated drugs.

The present invention will specifically be described in detail by way of Examples, but these Examples do not limit the scope of the present invention.

The conventionally used abbreviations indicated below are used for the listing of amino acid sequences of peptides contained in the Examples of the present invention. In these abbreviations, a single letter is used to represent an L-amino acid. The amino terminus (N terminus) of the peptide is listed first.

A: Alanine, R: Arginine, N: Asparagine, D: Aspartic acid, C: Cysteine, Q: Glutamine, E: Glutamic acid, G: Glycine, H: Histidine, I: Isoleucine, L: Leucine, K: Lysine, M: Methionine, F: Phenylalanine, P: Proline, S: Serine, T: Threonine, W: Tryptophan, Y: Tyrosine, V: Valine.

Example 1

In this Example, endothelin 1 was used as the biologically active peptide, and bovine type A receptor and rat type B receptor were used as the receptors.

Furthermore, the primary structure of Endothelin 1 is described in Japanese Unexamined Patent Publication No. 1-206997, etc., and is as indicated in Table 1.

[Table 1]

```
                    10                    20
      C S C S S L M D K E C V Y F C H L D I I W
```

1. Design of Endothelin 1 Complementary Peptide Using the Method of Blalock

A DNA strand was designed that was composed of a genetic codon able to code for Endothelin 1 in accordance with the method of Blalock. Next, the amino acid sequence specified by the associated DNA sequence was deduced from the 3' and 5' ends, respectively, of the genetic codon. The respective peptides were oriented from the amino terminus (N terminus) and carboxyl terminus (C terminus) that composed the peptides, and the complementary peptides indicated in Tables 2 through 5 below were designed according to the 4 design methods.

Furthermore, the abbreviations of the amino acids of the second row and below in each of the following tables indicate those amino acids that can be substituted for the amino acids in the first row.

[Table 2]

```
                            10                    20
      T G T G G K H I L L T N I K T V K I N N P
      A T A T T E   V F F A D V E A M E V D D
        R   R R Q           H         Q   Y Y
        A   A A             Y
      (Codon deducing direction: 5'→3', amino acid
      orientation direction: N → C)
```

[Table 3]

```
                            10                    20
      P N N I K V T K I N T L L I H K G G T G T
        D D V E M A E V D A F F V   E T T A T A
        Y Y   Q         H           Q R R   R
                        Y               A A   A
      (Codon deducing direction: 5'→ 3', amino acid
      orientation direction: C → N)
```

[Table 4]

```
                                 10                        20
        T R T R R N Y L F L T Q I K T V N L Y Y T
          S     S S E               H M           E
                    D                           D
```

(Codon deducing direction: 3'→ 5', amino acid orientation direction: N → C)

[Table 5]

```
                                 10                        20
        T Y Y L N V T K I Q T L F L Y N R R T R T
          E                       M H           E S S     S
          D                                     D
```

(Codon deducing direction: 3'→ 5', amino acid orientation direction: C → N)

2. Analysis of Homology Between Complementary Peptides and Receptors

An analysis was performed of the homology between the complementary peptides designed according to that described above and the respective primary amino acid sequences of bovine type A receptor and rat type B receptor. More specifically, respective partial sequences of 6 amino acids each were first prepared by moving one amino acid at a time from each of the amino terminuses in accordance with the amino acid sequences of the complementary peptides and receptors. The group of partial sequences of the complementary peptides and the group of partial sequences of the receptors were all compared, and those sites on each receptor at which there are 3 or more consecutive matching amino acids in the same order were identified. This identification was actually performed using a computer.

Those results are indicated in Table 6 and Table 7.

[Table 6]

EP 0 535 804 A1

Homology Analysis of Complementary Peptides to ET1 and Bovine Type A Receptor

```
                    ▼ 30                              60                              90                             120
METFWLRLSPWVALVGGVISDNPESYSTNLSIHVDSVATFHGTELSFVVTTHQPTNLALPSNGSMHNYCPQQTKITSAFKYINTVISCTIFIVGNVGNATLLRIIYQNKCNRNGPNALIA

                              150                             180                             210                            240
SLALGDLIYVVIDLPINVFKLLAGRWPPFEQNDFGVFLCKLFPFLQKSSVGITVLNLCALSVDRYRAVASWSRVQGIGIPLVTAIBIVSIWILSPILAIPEAIGFVKVPPFEYEGAQWRTCW

                              270                             300                             330                            360
LNATSKFWEFYQDVKDWWLFGFYFCNPLVCTAIFYTLWTCEWLNRRNGSLRIALSEHLKQRREVAKTVFCLVVIPALCWFPLHLSRILKKTYYDEWDTNRCELLSFLLLWDYIGINLATW

                              390                             420
WSCINPIALYFVSKKFKNCPQSCLCCCCYQSKSLWTSVPMNGTSIQWKNHEQNNHNTERSSHKDSIN
```

| | |
|---|---|
| ▬▬▬▬ | : Sugar chain binding site |
| ～～～～ | : Extracellular region |
| ───── | : Sequence matching 5' → 3' deduced, N → C synthesized complementary peptide |
| ·············· | : Sequence matching 5' → 3' deduced, C → N synthesized complementary peptide |
| ─ ─ ─ ─ | : Sequence matching 3' → 5' deduced, N → C synthesized complementary peptide |
| ─·─·─·· | : Sequence matching 3' → 5' deduced, C → N synthesized complementary peptide |

[Table 7]

Homology Analysis of Complementary Peptides to ET1 and Rat Type B Receptor

```
            ▼ 30                          60                          90                         120
MQSSASRCGRALVALLLACGLLGVWGEKRGFPPAQATPSLLGTKEVMTPPTKTSWTRGSNSSLNRFRTAEVTKGGRVAGVPPRSFPPPCQRKIEINKTFKYINTIVSCLVFVLGIIGNST

            150                         180                         210                         240
LLRIIYKNKCMRNGPNILIASLALGDLLHIIIDIPINAYKLLAGDWPFGAEMCKLVPFIQKASVGITVLSLCALSIDRYRAVASWSRIKGIGVPKWTAVEIVLIWVVSYVLAVPEAIGPD

            270                         300                         330                         360
VITSDYKGKPLRVCMLNPFQKTAFNQFYKTAKDWWLFSFYFCLPLAITAIFYTLNTCEMLRKKSGMQIALNDHLKQRREVAKTVFCLVLVPALCWLPLHLSRILKLTLYDQSNPQRCELL

            390                         420
SFLLVLDYIGINMASLNSCINPIALYLVSKRFKNCFKSCLCCWCQTFBEKQSLEEKQSCLKFKANDHGYDNFRSSNKYSSS
```

──────── : Sugar chain binding site
∼∼∼∼∼∼∼∼ : Extracellular region
──────── : Sequence matching 5' → 3' deduced, N → C synthesized complementary peptide
·············· : Sequence matching 5' → 3' deduced, C → N synthesized complementary peptide
────── : Sequence matching 3' → 5' deduced, N → C synthesized complementary peptide
·──·──·── : Sequence matching 3' → 5' deduced, C → N synthesized complementary peptide

EP 0 535 804 A1

On the other hand, an example in which the sites on the complementary peptides that are homologous with each receptor were analyzed using a similar method, is indicated in Table 8. Furthermore, the complementary peptides indicated in Table 8 are the result of deducing the complementary DNA strand of a DNA strand that actually codes for endothelin 1.

[Table 8]

$3' \to 5'$ deducing, $N \to C$ orientation:

T R T R R D Y L F K T Q M K T V D L ● ● T

$3' \to 5'$ deducing, $C \to N$ orientation:

T ● ● L D V T K M Q T L F L Y D R R T R T

_____ : Sites having high degree of homology with bovine type A receptor

_____ : Sites having high degree of homology with rat type B receptor

● : Termination codon

The peptides that include the homologous sites indicated in Tables 6, 7 and 8 above are peptides that can be selected according to the design method of the present invention.

3. Synthesis of Selected Peptides and Evaluation of Binding Activity

Although the following indicates an example of synthesis of the selected peptides, the conventionally used abbreviations indicated below which represent L-amino acids with three letters are used for the abbreviations of the amino acids used in this description. In addition, the amino terminus (N terminus) of the peptides is described first.

Ala: Alanine, Arg: Arginine, Asn: Asparagine, Asp: Aspartic acid, Cys: Cysteine, Gln: Glutamine, Glu: Glutamic acid, Gly: Glycine, His: Histidine, Ile: Isoleucine, Leu: Leucine, Lys: Lysine, Met: Methionine, Phe: Phenylalanine, Pro: Proline, Ser: Serine, Thr: Threonine, Trp: Tryptophan, Tyr: Tyrosine, Val: Valine.

(1) Synthesis Example Using a Solid Phase Synthesizer

The hexapeptide obtained by the above-mentioned design method was synthesized in which the amino acid sequence is indicated as Lys-Arg-Gly-Phe-Pro-Pro.

Synthesis of the above-mentioned peptide was performed using the Fmoc-polyamide method with a Model 9050 Peptide Synthesizer (MilliGen) using an Fmoc-Pro-Pep Syn KA resin column (MilliGen, Fmoc stands for 9-fluororenylmethyloxycarbonyl) as a solid phase.

After equilibrating 2.2 g of said solid phase resin (0.09 meq/g) column with dimethylformamide at a flow rate of 5 ml/min. for 1 minute, the Fmoc groups were removed with 20% piperidine/dimethylformamide (volume/volume) at a flow rate of 5 ml/min. for 10 minutes. Said resin was then washed with dimethylformamide at a flow rate of 5 ml/min. for 15 minutes. Subsequently, 4-fold amount (moles) of amino -acids bound to the surface of the solid phase of a pentafluorophenyl ester of Fmoc-Pro (Opfp) were dissolved in 2.64 ml of 5% (weight/volume) of 1-hydroxybenzotriazole (HOBT)/dimethylformamide and circulated on said resin column for 30 minutes to perform elongation reaction of the amino acids. Following the reaction, said resin column was washed with dimethylformamide, after which the resin column was then used in the next stage. Roughly 1 hour was required for the first stage reaction described above.

Moreover, after the above-mentioned cycle was repeatedly reacted using the pentafluorophenyl esters of the protective amino acids, namely Fmoc-Phe, Fmoc-Gly, Fmoc-Arg (Mtr) and Fmoc-Lys(Boc), in that order at 4-fold amount (moles) of the amino acids bound on the solid phase surface to be described next, the peptide resin, namely Lys(Boc)Arg(Mtr)-Gly-Phe-Pro-Pro-Pep Syn KA resin, was obtained after removing the Fmoc groups by washing with 20% piperidine/dimethylformamide at a flow rate of 5 ml/min. for 10 minutes, dimethylformamide at an equal flow rate for 15 minutes, and finally dichloromethane at an equal flow rate for 15 minutes.

It should be noted that removal of Fmoc groups following elongation of 2 amino acids was performed for 1 hour with 0.2% piperidine/dimethylformamide (volume/volume).

The peptide resin obtained by the above-mentioned reaction was taken out of the column, 0.6 ml of m-cresol, and 3.6 ml of thioanisol and 20.0 ml of trifluoroacetic acid were added thereto, followed by stirring for 1 hour at room temperature to elute the peptide from the resin. The eluents were collected as filtrate, followed by the addition of 1.8 ml of ethanedithiol and 4.0 ml of trimethylbromosilane to said filtrate, stirring for 1 hour at 0°C and blowing in nitrogen gas. Next, 100 ml of ether was added to said mixture while stirring to precipitate the peptide followed by collection of the peptide by centrifugation for 5 minutes at 3,000 x g. After washing the precipitate collected by the above-mentioned procedure by centrifuging 4 times in ethanol, the precipitate was dried under reduced pressure to obtain the above-mentioned peptide.

The peptide obtained in this manner demonstrated a retention time of 29 minutes in HPLC using a 0.1 % aqueous solution of trifluoroacetic acid and acetonitrile (5%-23% gradient, 15 minutes, 0.5 ml/min.) on YMC-ODS-5 (diameter 4.6 mm x 150 mm, Yamamura Chemical).

The peak fraction having a retention time of 29 minutes obtained by injecting a 10 μg/5 μl aqueous solution of the peptide was placed in a test tube, and after drying under reduced pressure, the peptide was hydrolized for 1 hour at 150°C by vaporizing under reduced pressure (500 mTorr) 1% phenol/6N hydrochloric acid (volume/volume) in a PICO TAG workstation (Waters) reaction bath. Next, the wet peptide was dried under reduced pressure, and after dissolving in 10 μl of a mixed solution of ethanol, water and triethylamine in a blending ratio of 2 : 2 : 1 (volume:volume:volume), the solution was again dried under reduced pressure. Then, 20 μl of a mixed solution of ethanol, triethylamine, water and phenylisothiocyanate in a blending ratio of 7 : 1 : 1 : 1 (volume:volume:volume: volume) were added and allowed to react for 25 minutes at room temperature. As a result of separating the amino acids by HPLC using 50 μl of PTC-amino acid eluent A (Wako Pure Chemical Industries)/PTC-amino acid eluent B (Wako Pure Chemical Industries) (PTC-amino acid eluent B ratio: 0-70% gradient, 15 minutes, 1 ml/min.) for the developing solution, and determining the amino acid composition of said synthesized peptide from the peak heights of amino acid standards treated in the same manner, the peptide composition was found to be Lys 0.96(1), Arg 0.78(1), Gly 1.13(1), Phe 0.97(1) and Pro 2.15(2).

(2) Synthesis by Pin Technology for Evaluation of Peptide Binding Activity

The amounts of amino acids, amounts of reagents and pin arrangements required for peptide synthesis were calculated using software of Cambridge Research Biochemical Ltd. (CRB) on an NEC Model APC H5020 computer following the PT-02-3000 manual of CRB.

Peptide synthesis was performed using the pins, blocks and Fmoc(9-fluororenylmethyloxycarbonyl)-L-amino acids contained in the Mimotope Design Kit of CRB following the above-mentioned manual and booklet (Geysen, H.M.: Use of peptide synthesis to probe viral antigens for epitopes to a resolution of a single amino acid, Proc. Natl. Acad. Sci. U.S.A., 81, 3998-4002, (1984)). All of the amino acids with the exception of serine and threonine were pentafluorophenyl (-Opfp) esters in which the amino terminuses were protected with Fmoc groups, while serine and threonine were dihydroxybenzotriazole(-ODhbt) esters in which the amino terminuses were protected with Fmoc groups. In addition, the side chain protective groups for serine, threonine and tyrosine were t-butyl ether (-tBu) groups, the side chain protective groups for aspartic acid and glutamic acid were t-butyl ester (-OtBu) groups, the side chain protective groups for lysine and histidine were t-butoxycarbonyl (Boc-) groups, the side chain protective group for arginine was a 4-methoxy-2,3,6-trimethylbenzenesulfonyl (Mtr-) group, and the side chain protective group for cysteine was a trityl (Trt-) group. In addition, the pins used for synthesis had Fmoc-b alanine (bAla) bound to a polyethylene support, in which acrylic acid was grafted on the surface, while using hexamethylenediamine (HMD) as the spacer.

A specific example of this synthesis will be given below.

Synthesis of the Peptide Indicated by Lys-Arg-Gly-Phe-Pro-Pro

The 9-fluorenylmethyloxycarbonyl (Fmoc) groups of a resin, in which acrylic acid was grafted onto the pin (diameter: 4 mm) on the end of a polyethylene rod, and β-alanine, in which α-amino acids were protected with Fmoc groups by means of hexamethylenediamine (HMD) groups, was bound, were removed with 20% piper- idine/dimethlyformamide (volume/volume) for 30 minutes. After sequentially washing with excess amounts of dimethylformamide and methanol, the resin was air-dried. Moreover, after equilibrating the resin in dimethylformamide, the resin was immersed overnight at 30°C in 100 μl of 20 mM of pentafluorophenyl ester (-Opfp) of Fmoc-Pro dissolved in 22.5 mM 1-hydroxybenzotriazole (HOBT)/dimethylformamide. The resin was then sequentially washed with excess amounts of dimethylformamide and methanol to obtain the Fmoc-Pro-bAla-HMD resin. Next, the protective groups (Fmoc groups) of the α-amino acid groups of said peptide resin were removed by the above-mentioned treatment. Moreover, the reactions of the above-mentioned cycle were repeated using 20 mM pentafluorophenyl esters of the protected amino acids, namely Fmoc-Pro, FmocPhe, Fmoc-Gly, Fmoc-Arg(Mtr) and Fmoc-Lys(Boc), in this order to obtain the protected peptide resin, namely Fmoc-Lys (Boc)-Arg(Mtr)-Gly-Phe-Pro-Pro-bAla-HMD resin.

The protective groups (Fmoc groups) of the α-amino acids of said peptide resin were removed by the above-mentioned treatment, and the resin was air-dried after sequentially washing with excess amounts of dimethylformamide and methanol. An acetylation reaction was performed for 90 minutes at 30°C by immersing said peptide resin in a mixed solution of dimethylformamide, anhydrous acetic acid and triethylamine in the ratio of 5 : 2 : 1 (volume:volume: volume). After similarly sequentially washing the resin with excess amounts of dimethylformamide and methanol, the resin was air-dried. Next, said peptide resin was immersed in a mixed solution of trifluoroacetic acid, anisole and ethanedithiol in a ratio of 95 : 2.5 : 2.5 (volume:volume:volume) and allowed to react for 4 hours at room temperature. After removing the protective groups and air-drying, said peptide was subjected to adequate ultrasonic washing in a mixture of 0.1% hydrochloric acid (volume/volume) and 50% (volume/volume) methanol to obtain the Ac-Lys-Arg-Gly-Phe-Pro-Pro-bAla-HMD resin.

(3) Measurement of Endothelin 1 Binding Activity of the Peptide Synthesized Above

a: [Enzymeimmuno Competitive Inhibition Assay Method]

After blocking those sites susceptible to nonspecific adsorption on the peptide resin obtained in (2) above with 0.2% skim milk solution, the peptide resin was immersed in 200 μl of human endothelin 1 solution (0.2 μg/ml) (prepared by dissolving in Dalbecco's phosphate buffered biological saline containing 0.1% skim milk and 0.1% Tween 20). After allowing to react for 3 hours at room temperature while stirring gently, the resulting solution was used as sample solution (i).

Separate from the above, the wells of an ELISA microplate were coated overnight at 4°C with 200 μl of human endothelin 1 solution (1 μg/ml). Moreover, the uncoated sites on the plate were blocked with 0.2% skim milk solution on the following day to prepare the human endothelin 1 coated plate (ii).

After washing the human endothelin 1 coated plate (ii) with washing buffer (Dalbecco's phosphate buffered biological saline containing 0.1% Tween), 50 μl of sample solution (i) was added followed immediately by the addition of 200 μl of anti-human endothelin 1 rabbit antibody solution (using Dalbecco's phosphate buffered biological saline containing 0.1% skim milk and 0.1% Tween as the dilution buffer). The competition reaction was then performed for 2 hours at room temperature. Following said competition reaction, the plate was thoroughly washed with washing buffer followed by the addition of 200 μl of alkaline phosphatase-labelled anti-rabbit IgG, A, M goat antibody solution diluted 1 : 2,000. The binding reaction was then allowed to take place for 2 hours at room temperature with the anti-human endothelin 1 rabbit antibody captured on the plate. After similarly washing the plate and adding 200 μl of substrate solution (1 mg p-nitrophenyl phosphate/ml 1M diethanolamine buffer), the amount of p-nitrophenol released by the labelled enzyme present on the plate was determined by measuring the absorbance at 405 nm. The amount of human endothelin 1 in sample solution (i) was then calculated from a separately prepared calibration curve.

As a result, the antigen activity of human endothelin 1 in said sample solution (i) decreased to 78.9% prior to performing the binding reaction to the peptide resin pins (inhibitory activity: 21.1%).

b: [Binding Activity of Radioactive Ligand]

After blocking the sites susceptible to adsorption on the peptide resin obtained in (2) above with 0.2% skim milk solution, the peptide was suspended in 100 ml of [125]I-human endothelin 1 (specific activity: 74 TBq-/mmol) solution (0.131 ng/ml) (prepared by dissolving in Dalbecco's phosphate buffered biological saline containing 0.1% skim milk and 0.1% Tween) and allowed to react for 2 hours at room temperature. Following the binding reaction, the peptide resin was thoroughly washed with washing buffer (Dalbecco's phosphate buffered biological saline containing 0.1% Tween), and the radioactivity bound to the peptide resin was measured with a gamma counter.

As a result, the amount of human endothelin 1 bound to the peptide resin was calculated to be 5.18 pg.

In the method described in (2) above, numerous types of peptides selected according to the design method of the present invention were synthesized, and the binding activity of those peptides was evaluated according to the method described in (3) above. Those results are indicated in Table 9.

[Table 9]

Table 9

| Synthesized Peptides | | Enzymeimmuno Competitive Inhibition Activity (%) | Radiactive Ligand Binding Activity (pg) |
|---|---|---|---|
| Bovine Type A Receptor Origin | TKITSA | 3.9 | 1.880 |
| | VFLCKL | 2.0 | 1.197 |
| | KGAQHR | 8.1 | 3.015 |
| Rat Type B Receptor Origin | KRGFPP | 21.1 | 5.176 |
| | AEVTKG | 3.0 | 1.264 |
| | VTKGGR | 8.5 | 3.454 |
| | KGGRVA | 6.7 | 2.501 |
| | GAEMCK | 5.9 | 1.723 |
| | SDYKGK | 17.0 | 4.248 |
| | KTAFMQ | 4.7 | 0.938 |
| | REVAKT | 10.8 | 3.714 |
| | VAKTVF | 3.0 | 2.232 |
| Complementary Peptide Origin | VTKMQT | 14.0 | 1.935 |
| | QMKTVD | 9.1 | 0.278 |

Example 2

In this example, diuretic hormone was used for the biologically active peptide, and type A receptor, type B receptor and type C receptor were used for the receptors.

The primary structure of diuretic hormone is as indicated in Table 10.

[Table 10]

```
                          10                        20
      S  L  R  R  S  S  C  F  G  G  R  M  D  R  I  G  A  Q  S  G  L  G  C  N  S  F  R  Y
```

1. Design of Diuretic Hormone Complementary Peptide Using the Method of Blalock

A DNA strand was designed comprising a genetic codon able to code for diuretic hormone in accordance with the method of Blalock. Next, the amino acid sequence specified by the complementary DNA sequence was deduced from the 3′ and 5′ terminuses, respectively, of the genetic codon. The respective peptides were oriented from the amino terminus (N terminus) and carboxyl terminus (C terminus) that compose the peptides, and the four peptides indicated in Tables 11 through 14 below were designed according to the design method.

Incidentally, the abbreviations of the amino acids of the second row and below in each of the following tables indicate those amino acids that can be substituted for the amino acids in the first row.

[Table 11]

```
                         10                      20
    G K A A G G T K T T A H I A N T R L G T K T T I G K A I
    T E T T T T A E S S   V T D S S   T S E S A V T E T V
    R Q S S R R   P P S   S Y P G   R P Q P   R   S
    A   P P A A   A A P   P   A C   A A   A   A   P
```

(Codon deducing direction: 5' → 3', amino acid orientation direction: N → C)


[Table 12]

```
                         10                      20
    I A K G I T T K T G L R T N A I H A T T K T G G A A K G
    V T E T V A S E S T   S S D T V   T S S E A T T T T E T
    S   R   P Q P R   G P Y S   S P P   R R S S Q R
    P   A   A A A   C A P   P A A   A A P P   A
```

(Codon deducing direction: 5' → 3', amino acid orientation direction: C → N)


[Table 13]

```
                         10                      20
    R N A A R R T K P P A Y L A Y P R V R P N P T L R K A I
    S E S S S S     S   S     S   E     S   S M
    D                               D
```

(Codon deducing direction: 3' → 5', amino acid orientation direction: N → C)


[Table 14]

```
                         10                      20
    I A K R L T P N P R V R P Y A L Y A P P K T R R A A N R
    M S   S     E   S     S   S       S S S S E S
                    D                             D
```

(Codon deducing direction: 3' → 5', amino acid orientation direction: C → N)


2. Analysis of Homology Between Complementary Peptides and Receptors

An analysis was performed of the homology between the complementary peptides designed according to that described above and the respective primary amino acid sequences of type A receptor, type B receptor and type C receptor. More specifically, respective partial sequences of 6 amino acids each were first prepared by moving one amino acid at a time from each of the amino terminuses in accordance with the amino acid sequences of the complementary peptides and receptors. The group of partial sequences of the complementary peptides and the group of partial sequences of the receptors were all compared, and those sites on each receptor at which there are 4 or more consecutive matching amino acids in the same order were analyzed. In addition, an analysis of the homology between the respective primary amino acid sequences of type A receptor, type B receptor and type C receptor was performed in the same manner as above for complementary peptides designed based only on a genetic codon that actually codes for the amino acid sequence of diuretic hormone. This analysis was performed for sites at which there are not 4 or more, but rather at least 3 consecutive matching amino acids in the same order. This analysis was actually performed using a computer.

Those results are indicated in Tables 15, 16 and 17.

[Table 15]

EP 0 535 804 A1

Homology Analysis of Complementary Peptides to ANP and Type A Receptor

MPGPRRPAGSRLRLLLLLLLPPLLLLLRGSHAGNLTVAVVLPLANTSYPWSWARVGPAVELALAQVKARPDLLPGHTVRTYLGSSENALGVCSDTAAPLAAVDLKWEHNPAVFLGPGCVY

AAAPVGRPTAHWRVPLLTAGAPALGPGVKDEYALTTRAGPSYAKLGDFVAALIIRRLGWERQALMLYAYRPGDEEHCFFLVEGLFMRVRDRLNITVDIILEFAEDDLSHYTRLLRTNPRKGR

VIYICSSPDAPRTLMLLALEAGLCGEDYVFFIILDIFGQSLQGGQGPAPRRPWERGDCQDVSARQAPQAAKIITYKDPDNPEYLEPLKQLKHLAYEQFNPTMEDGLVNTIPASPHDGLLLY

IQAVTETLAIIGGTVTDGENITQRMWNRSPQGVTGYLKIDSSGDRETDPSLWDMDPENGAFRVVLNYNGTSQELVAVSGRKLNWPLGYPPPDIPKCGFDNEDPACNQDHLSTLB······  → Within membrane

| | |
|---|---|
| ———————— | : Sugar chain binding site |
| ∼∼∼∼∼∼∼∼ | : β-turn or coil structure |
| ———————— | : Sequence matching 5' → 3' deduced, N → C synthesized complementary peptide (originating in a nucleic acid sequence coding for ANP) |
| ·················· | : Sequence matching 5' → 3' deduced, C → N synthesized complementary peptide (originating in a nucleic acid sequence coding for ANP) |
| - - - - - - - | : Sequence matching 3' → 5' deduced, N → C synthesized complementary peptide (originating in a nucleic acid sequence coding for ANP) |
| ———————— | : Sequence matching 5' → 3' deduced, N → C synthesized complementary peptide (originating in a nucleic acid sequence able to code for ANP) |
| ·················· | : Sequence matching 5' → 3' deduced, C → N synthesized complementary peptide (originating in a nucleic acid sequence able to code for ANP) |

[Table 16]

EP 0 535 804 A1

Homology Analysis of Complementary Peptides to ANP and Type B Receptor

```
                  ▼   30                              60                              90                          120
MALPSLLLLVAALAGGVRPPGARNLTLAVVLPEHNLSYAWAWPRVGPAVALAVEALGRALPVDLRFYSSELEGACSEYLAPLSAVDLKLYHDPDLLLGPGCYYPAASVARPASHWRLPLL


                           150                             180                             210                         240
TAGAVASGFSAKNDHYRTLVRTGPSAPKLGEFVVTLHGHFNRTARAALLYLDARTDDRPHYFTIEGVPEALQGSNLSVQHQVYAREPGGPEQATHFIRANGRIVYICGPLEWLHEILLQA


                           270                             300                             330                         360
QRENLTHGDYVFFYLDVFGESLRAGPTRATGRPWQDNRTREQAQALREAPQTVLVITYREPPHPEYQEFQNRLLIRAREDPGVELGPSLWNLIAGCFYDGILLYAEVLNRTIQEGGTRED


                           390                             420                             450        → Within membrane
GLRIVEKMQGRRYHGVTGLVVMDKNNDRETDFVLWAMGDLDSGDPQPAAHYSGAEKQIWWTGRPIPWVKGAPPSDNPPCAFDLDDPSCDKTPLST·······················
```

———————— : Sugar chain binding site
∿∿∿∿∿∿∿∿ : β-turn or coil structure
———————— : Sequence matching 5' → 3' deduced, N → C synthesized complementary peptide
            (originating in a nucleic acid sequence coding for ANP)
················· : Sequence matching 5' → 3' deduced, C → N synthesized complementary peptide
            (originating in a nucleic acid sequence coding for ANP)
— — — — : Sequence matching 3' → 5' deduced, N → C synthesized complementary peptide
            (originating in a nucleic acid sequence coding for ANP)
·—·—·—· : Sequence matching 3' → 5' deduced, C → N synthesized complementary peptide
            (originating in a nucleic acid sequence coding for ANP)
———————— : Sequence matching 5' → 3' deduced, N → C synthesized complementary peptide
            (originating in a nucleic acid sequence able to code for ANP)
················· : Sequence matching 5' → 3' deduced, C → N synthesized complementary peptide
            (originating in a nucleic acid sequence able to code for ANP)

[Table 17]

EP 0 535 804 A1

Homology Analysis of Complementary Peptides to ANP and Type C Receptor

```
             30               ▼            60                          90                        120
MPSLLVLTFSPCVLLGWALLAGGTGGGGVGGGGGGAGIGGGRQEREALPPQKIEVLVLLPQDDSYLFSLTRVRPAIEYALRSVEGNGTGRRLLPPGTRFQVAFEDSDCGNRALFSLVDRV

             150              180                         210                       240
AAARGAKPDLILGPVCEYAAAPVARLASHWDLPWLSAGALAAGFQHKDSEYSHLTRVAPAYAKNGENWLALFRJIHHWSRAALVYSDDKLERNCYFTLEGVHEVFQEEGLHTSIYSFDETK

             270              300                         330                       360
DLDLEDIVRNIQASERVYINCASSDTIRSINLVAHRHGNTSGDYAFFNIELFNSSSYGDGSWKRGDKIIDFEAKQAYSSLQTVTLLRTVKPEFBKFSNEVKSSVEKQGLNMEDYVNNFVEG

                                                                                    Within membrane
             390              420                         450                       480→
FHDAILLYVLALHEVLRAGYSKKDGGKIIQQTWNRTFEGIAGQVSIDANGDRYGDFSVIANTDVEAGTQEVIGDYPGKEGRFENRPNVKYPWGPLKLRIDENRIVEHTNSSPCKSSGGLE
```

———————— : Sugar chain binding site

〰〰〰〰 : β-turn or coil structure

⋯⋯⋯⋯ : Sequence matching 5' → 3' deduced, C → N synthesized complementary peptide
(originating in a nucleic acid sequence coding for ANP)

·—————— : Sequence matching 3' → 5' deduced, N → C synthesized complementary peptide
(originating in a nucleic acid sequence coding for ANP)

·—·—·—·— : Sequence matching 3' → 5' deduced, C → N synthesized complementary peptide
(originating in a nucleic acid sequence coding for ANP)

———————— : Sequence matching 5' → 3' deduced, N → C synthesized complementary peptide
(originating in a nucleic acid sequence able to code for ANP)

·⋯⋯⋯⋯ : Sequence matching 5' → 3' deduced, C → N synthesized complementary peptide
(originating in a nucleic acid sequence able to code for ANP)

·—————— : Sequence matching 3' → 5' deduced, N → C synthesized complementary peptide
(originating in a nucleic acid sequence able to code for ANP)

·—·—·—·— : Sequence matching 3' → 5' deduced, C → N synthesized complementary peptide
(originating in a nucleic acid sequence able to code for ANP)

The peptides that include the homologous sites indicated in Tables 15, 16 and 17 above are peptides that can be selected according to the design method of the present invention.

3. Synthesis of Selected Peptides and Evaluation of Binding Activity

Several peptides were synthesized according to the pin technology method described in Example 1 to evaluate the an activity to bind to of the peptides selected using the above-mentioned method with respect to diuretic hormone. These peptides were then evaluated in the same manner as the method described in Example 1. Those results are indicated in Table 18.

Table 18

| Synthesized Peptides | | Enzeimmuno Competitive Inhibition Activaty (%) | Radioactive Ligand Binding Activity (pg) |
|---|---|---|---|
| Type A Receptor Origin | GVCSDT | 15.3 | 0.845 |
| | CSDTAA | 13.9 | 0.460 |
| | TAGAPA | 13.6 | 0.391 |
| | SPDAFR | 12.6 | 0.451 |
| | EAGLCG | 9.8 | 0.544 |
| Type B Receptor Origin | REPPNP | 18.1 | 0.638 |
| | DPSCDK | 18.1 | 0.413 |
| Type C Receptor Origin | VRPAIE | 20.0 | - |
| | IEYALR | 31.0 | 0.971 |

Effect of Invention

Peptides that are designed and produced according to the method of the present invention have specific reactivity and a high degree of an activity to bind to biologically active peptides. Thus, the resulting peptides are extremely effective in the designing of drugs, including detection reagents used in the measurement of said biologically active peptides and all or some of their precursors, and therapeutic drugs that inhibit or modify the functions of said biologically active peptides.

Sequence Listing


Sequence number: 1
Length of sequence: 6
Form of sequence: amino acid
Topology: straight chain
Type of sequence: peptide
Sequence: Thr  Lys  Ile  Thr  Ser  Ala
                 1                5


Sequence number: 2
Length of sequence: 6
Form of sequence: amino acid
Topology: straight chain
Type of sequence: peptide
Sequence: Val  Phe  Leu  Cys  Lys  Leu
                 1                5


Sequence number: 3
Length of sequence: 6
Form of sequence: amino acid
Topology: straight chain
Type of sequence: peptide
Sequence: Lys  Gly  Ala  Gln  His  Arg
                 1                5


Sequence number: 4
Length of sequence: 6
Form of sequence: amino acid
Topology: straight chain
Type of sequence: peptide
Sequence: Lys  Arg  Gly  Phe  Pro  Pro
                 1                5

Sequence number: 5

Length of sequence: 6

Form of sequence: amino acid

Topology: straight chain

Type of sequence: peptide

Sequence: Ala   Glu   Val   Thr   Lys   Gly
            1                5


Sequence number: 6

Length of sequence: 6

Form of sequence: amino acid

Topology: straight chain

Type of sequence: peptide

Sequence: Val   Thr   Lys   Gly   Gly   Arg
            1                5


Sequence number: 7

Length of sequence: 6

Form of sequence: amino acid

Topology: straight chain

Type of sequence: peptide

Sequence: Lys   Gly   Gly   Arg   Val   Ala
            1                5


Sequence number: 8

Length of sequence: 6

Form of sequence: amino acid

Topology: straight chain

Type of sequence: peptide

Sequence: Gly   Ala   Glu   Met   Cys   Lys
            1                5

Sequence number: 9
Length of sequence: 6
Form of sequence: amino acid
Topology: straight chain
Type of sequence: peptide
Sequence: Ser  Asp  Tyr  Lys  Gly  Lys
               1                5


Sequence number: 10
Length of sequence: 6
Form of sequence: amino acid
Topology: straight chain
Type of sequence: peptide
Sequence: Lys  Thr  Ala  Phe  Met  Gln
               1                5


Sequence number: 11
Length of sequence: 6
Form of sequence: amino acid
Topology: straight chain
Type of sequence: peptide
Sequence: Arg  Glu  Val  Ala  Lys  Thr
               1                5


Sequence number: 12
Length of sequence: 6
Form of sequence: amino acid
Topology: straight chain
Type of sequence: peptide
Sequence: Val  Ala  Lys  Thr  Val  Phe
               1                5

Sequence number: 13
Length of sequence: 6
Form of sequence: amino acid
Topology: straight chain
Type of sequence: peptide
Sequence: Gln  Met  Lys  Thr  Val  Asp
             1               5


Sequence number: 14
Length of sequence: 6
Form of sequence: amino acid
Topology: straight chain
Type of sequence: peptide
Sequence: Val  Thr  Lys  Met  Gln  Thr
             1               5


Sequence number: 15
Length of sequence: 6
Form of sequence: amino acid
Topology: straight chain
Type of sequence: peptide
Sequence: Gly  Val  Cys  Ser  Asp  Thr
             1               5


Sequence number: 16
Length of sequence: 6
Form of sequence: amino acid
Topology: straight chain
Type of sequence: peptide
Sequence: Cys  Ser  Asp  Thr  Ala  Ala
             1               5

Sequence number: 17
Length of sequence: 6
Form of sequence: amino acid
Topology: straight chain
Type of sequence: peptide
Sequence: Thr  Ala  Gly  Ala  Pro  Ala
             1                 5


Sequence number: 18
Length of sequence: 6
Form of sequence: amino acid
Topology: straight chain
Type of sequence: peptide
Sequence: Ser  Pro  Asp  Ala  Phe  Arg
             1                 5

Sequence number: 19
Length of sequence: 6
Form of sequence: amino acid
Topology: straight chain
Type of sequence: peptide
Sequence: Glu  Ala  Gly  Leu  Cys  Gly
             1                 5


Sequence number: 20
Length of sequence: 6
Form of sequence: amino acid
Topology: straight chain
Type of sequence: peptide
Sequence: Arg  Glu  Pro  Pro  Asn  Pro
             1                 5

```
Sequence number: 21
Length of sequence: 6
Form of sequence: amino acid
Topology: straight chain
Type of sequence: peptide
Sequence: Asp  Pro  Ser  Cys  Asp  Lys
             1                 5


Sequence number: 22
Length of sequence: 6
Form of sequence: amino acid
Topology: straight chain
Type of sequence: peptide
Sequence: Val  Arg  Pro  Ala  Ile  Glu
             1                 5


Sequence number: 23
Length of sequence: 6
Form of sequence: amino acid
Topology: straight chain
Type of sequence: peptide
Sequence: Ile  Glu  Tyr  Ala  Leu  Arg
             1                 5
```

## Claims

1. A method for designing a peptide having an activity to bind to a biologically active peptide (Peptide A) which comprises the steps of:
   a) determining the amino acid sequence (Sequence R) of a receptor of Peptide A;
   b) deducing a DNA strand (Strand C) complementary to a DNA strand comprising a genetic coding sequence able to code for Peptide A;
   c) determining an amino acid sequence (Sequence C) of a peptide complementary to Peptide A based on Strand C;
   d) comparing homology of Sequence R and Sequence C; and
   e) selecting from Sequence R or Sequence C a partial amino acid sequence consisting of at least more than 6 amino acid residues in which a number of matching amino acids in the same order is equal to or greater than a number of non matching amino acids.

2. The method for designing the peptide according to claim 1 wherein Sequence C is determined by deducing Strand C from the 5′ end of the genetic coding sequence of Strand C and by arranging amino acids starting from an N terminus side.

3. The method for designing the peptide according to claim 1 wherein Sequence C is determined by deducing Strand C from the 5′ end of the genetic coding sequence of Strand C and by arranging amino acids starting from a C terminus side.

4. The method for designing the peptide according to claim 1 wherein Sequence C is determined by deducing Strand C from the 3′ end of the genetic coding sequence of Strand C and by arranging amino acids starting from an N terminus side.

5. The method for designing the peptide according to claim 1 wherein Sequence C is determined by deducing Strand C from the 3′ end of the genetic coding sequence of Strand C and by arranging amino acids starting from a C terminus side.

6. The method for designing the peptide according to claims 1-5 wherein the genetic coding sequence able to code for Peptide A is identical with the coding sequence for Peptide A.

7. A method for designing a peptide having an activity to bind to Peptide A comprising the additional step of selecting a partial amino acid sequence located at surface of cells of the receptor from the partial amino acid sequences obtained by the method according to claim 1.

8. A method for designing a peptide having an activity to bind to Peptide A comprising the additional step of selecting a partial amino acid sequence having a β-turn or coil structure of the receptor from the partial amino acid sequences obtained by the method according to claim 1.

9. A method for designing a peptide having an activity to bind to Peptide A comprising the additional step of selecting a partial amino acid sequence having no glycosylation site of the receptor from the partial amino acid sequences obtained by the method according to claim 1.

10. A method for designing a peptide having an activity to bind to Peptide A comprising the additional step of selecting a partial amino acid sequence common to peptides designed by at least two methods of claims 2-5 from the partial amino acid sequences obtained by the method according to claim 1.

11. A method for designing a peptide having an activity to bind to Peptide A which comprises reversing the partial amino acid sequence obtained by the method according to claim 1 in the N terminus side and C terminus side.

12. A method for producing a peptide having an activity to bind to Peptide A which comprises designing a peptide by the method according to any one of claims 1-11 and producing a peptide containing a consecutive sequence of at least 4 amino acids in the designed peptide.

13. A peptide having an activity to bind to Peptide A or a salt thereof produced by the method of claim 12.

14. The peptide according to claim 13 wherein the peptide is optionally blocked or protected at the N terminus, C terminus or both terminuses.

15. The peptide and its salt having at least 4 consecutive amino acid sequences each selected from the amino acid sequences of TKITSA, VFLCKL KGAQHR, ASTIKT, LKCLFV, RHQAGK, KRGFPP, AEVTKGGRVA, GAEMCK, SDYKGK, KTAFMQ, REVAKTVF, FVTKAVER, QMFATK, KGKYDS, KCMEAG, AVRGGKTVEA, PPFGRK, DVTKMQT AND TQMKTVD.

16. The peptide and its salt and having at least 4 consecutive amino acid sequences each selected from the amino acid sequences of GVCSDTAA, TAGAPA, SPDAFR, EAGLCG, GCLGAE, RFADPS, APAGAT, AATDSCVG, REPPNP, DPSCDK, KDCSPD, PNPPER, VFPAIEYALR and RLAYEIAPRV.

17. A method for determining a binding site of Peptide A on a receptor of Peptide A which comprises the steps of:
    a) determining an amino acid sequence of the receptor of Peptide A;
    b) deducing a DNA strand complementary to a DNA strand comprising genetic codes able to code for Peptide A;
    c) determining an amino acid sequence of a peptide complementary to Peptide A based on Strand C;
    d) comparing homology of Sequence R and Sequence-C; and
    e) selecting from Sequence R the partial amino acid sequence consisting of at least more than 6 amino acid residues in which a number of amino acids matching in the same order in Sequence R and Sequence C is equal to or greater than a number of not matching amino acids.

18. A diagnostic reagent for detecting Peptide A containing a peptide according to any one of claims 13 to 16.

19. A pharmaceutical agent able to bind to Peptide A comprising a peptide as claimed in any one of claims 13 to 16.

20. An agent as claimed in claim 19 wherein Peptide A is endothelin and said peptide is as defined in claim 15 or wherein Peptide A is diuretic hormone and said peptide is as defined in claim 16.

**European Patent Office**

## EUROPEAN SEARCH REPORT

Application Number

| | DOCUMENTS CONSIDERED TO BE RELEVANT | | EP 92307976.8 |
|---|---|---|---|

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 108, no. 13, March 28, 1988, Columbus, Ohio, USA SHAI YECHIEL et al. "Anti-sense peptides - designing recognition molecules" page 240, column 2, abstract-no. 108 206j & UCLA Symp.Mol.Cell.Biol., New Ser. 1987, 69(Protein Struct.,Folding,Des.2), 439-51 -- | 1-12 | C 07 K 1/00<br>C 07 K 7/06<br>G 01 N 33/74<br>A 61 K 37/02<br>A 61 K 37/43<br>C 12 Q 1/68 |
| X | WO - A - 86/05 208 (UNIVERSITY OF TEXAS SYSTEM) * Claims 1-4,8-11,20-22,67-70 * -- | 1-20 | |
| A | CHEMICAL ABSTRACTS, vol. 114, no. 23, June 10, 1991, Columbus, Ohio, USA SINGH JUSWINDER et al. "A novel method for the modeling of peptide ligands to their receptors" page 400-01, column 2(1), abstract-no. 224 482u & Protein Eng. 1991,4(3), 251-61 -- | 1-12 | TECHNICAL FIELDS SEARCHED (Int. Cl.5)<br><br>C 07 K 1/00<br>C 07 K 7/00<br>G 01 N 33/00<br>A 61 K 37/00<br>C 12 Q 1/00 |
| A | CHEMICAL ABSTRACTS, vol. 99, no. 9, August 29, 1983, Columbus, Ohio, USA B. BUSETTA et al. "DOCKER, an interactive program for simulating protein receptor and receptor and substrate interactions" page 327-28, column 2(1), abstract-no. 67 113b | 1-12 | |

The present search report has been drawn up for all claims

| Place of search | Date of completion of the search | Examiner |
|---|---|---|
| VIENNA | 17-12-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | & J.Appl.Crystallogr. 1983, 16(4), 432-7 | | |

TECHNICAL FIELDS
SEARCHED (Int. Cl.5)

The present search report has been drawn up for all claims

| Place of search | Date of completion of the search | Examiner |
|---|---|---|
| VIENNA | 17-12-1992 | SCHARF |